# EUROPEAN PATENT SPECIFICATION

(11) **EP 1 104 289 B1**
(45) Date of publication and mention of the grant of the patent: **01.02.2006**
(21) Application number: 99942892.3
(22) Date of filing: 19.08.1999
(51) Int. Cl.: A61K 9/20, A61P 1/00

(54) **NEW ORAL FORMULATION FOR 5-HT4 AGONISTS OR ANTAGONISTS**
NEUE ORALE FORMULIERUNGEN FÜR 5-HT4 AGONISTEN ODER ANTAGONISTEN
NOUVELLE FORMULATION ORALE D'AGONISTES OU D'ANTAGONISTES DE 5-HT4

(30) Priority: 21.08.1998 GB 9818340; 27.10.1998 GB 9823477; 05.05.1999 GB 9910320; 12.05.1999 GB 9911059
(43) Date of publication of application: 06.06.2001
(73) Proprietor: Novartis AG, 4056 Basel (CH); Novartis Pharma GmbH, 1230 Wien (AT)
(72) Inventor: DE BRUIJN, Karel, F-68730 Blotzheim (FR); ENGEL, Günter, D-79576 Weil (DE); PFANNKUCHE, Hans-Jürgen, D-79576 Weil (DE); THEWISSEN, Michael, D-40227 Düsseldorf (DE); VITZLING, Christian, F-75012 Paris (FR); ZÜGER, Othmar, CH-4123 Allschwil (CH)
(74) Representative: de Weerd, Petrus G.W.
(86) International application number: PCT/EP1999/006083
(87) International publication number: WO 2000/010526

(56) References cited:
- EP-A- 0 505 322
- EP-A- 0 691 340
- EP-A- 0 732 333
- WO-A-96/16639
- WO-A-97/22335
- WO-A-98/03173
- FR-A- 2 753 196
- US-A- 5 399 562
- US-A- 5 523 289
- CHEMICAL ABSTRACTS, vol. 127, no. 15, 13 October 1997 (1997-10-13) Columbus, Ohio, US; abstract no. 210377, OUCHI, HIROSHI ET AL: "Digestive tract disease-treating agents" XP002129585 & JP 09 194374 A (TAISHO PHARMACEUTICAL CO., LTD., JAPAN) 29 July 1997 (1997-07-29)
- PATENT ABSTRACTS OF JAPAN vol. 013, no. 419 (C-637), 18 September 1989 (1989-09-18) & JP 01 156909 A (CHUGAI PHARMACEUT CO LTD), 20 June 1989 (1989-06-20)
- AMER. PHARM. ASSOC. / PHARM. SOC. OF GREAT BRITAIN: "Handbook of pharmaceutical excipients" 1988 , AM. PHARM. ASSOC. , WASHINGTON XP002129584 153140 page 289 -page 293
- STEINER A. ET AL: "Drugs coordinating and restoring gastrointestinal motility and their effects on selected hypodynamic gastrointestinal disorders to horses and cattle" J. OF VET. MED. SERIES A, vol. 42, 1995, pages 613-631, XP000910391
- BRIKAS P.: "Motor modifying properties of 5-HT3 and 5-HT4 receptor agonists on ovine abomasum " J. OF VET. MEDICINE SERIES A, vol. 41, 1994, pages 150-158, XP000910373
- GASTER L.: "SB207266 : 5-HT4 receptor antagonist agent for irritable bowel syndrome" DRUGS FUTURE, vol. 22, no. 12, 1997, pages 1325-1332, XP002099385
- LANGAKER KJ ET AL: "The partial 5-HT4 ( HTF 919 ) improves symptoms in constipation predominant irritable bowel syndrome ( C-IBS )" DIGESTION, vol. 59, no. 3, 6 September 1998 (1998-09-06), page gapp00064 XP000909235
- FARTHING M.: "New drugs in the management of the irritable bowel syndrome" DRUGS , vol. 56, no. 1, July 1998 (1998-07), pages 11-21, XP000909287
- BERKOW R. ET AL: "The Merck Manual of Diagnosis and Therapy" 1992 , MERCK RESEARCH LABORATORIES , USA XP002138260 215310 page 841 -page 842
- SCHUTZE K. ET AL: "Double blind study of the effect of cisapride on constipation and abdominal discomfort as component of the irritable bowel syndrome" ALIMENT. PHARMACOL. THER., vol. 11, no. 2, 1997, pages 387-394, XP000909210
- TALLEY N J: "REVIEW ARTICLE: 5-HYDROXYTRYPTAMINE AGONISTS AND ANTAGONISTS IN THE MODULATION OF GASTROINTESTINAL MOTILITY AND SENSATION: CLINICAL IMPLICATIONS" ALIMENTARY PHARMACOLOGY & THERAPEUTICS,GB,BLACKWELL SCIENTIFIC PUBLICATIONS LTD., CAMBRIDGE, vol. 6, no. 3, 1 June 1992 (1992-06-01), pages 273-289, XP000566133 ISSN: 0269-2813
- SCOTT & PERRY: "TEGASEROD" DRUGS,AT,ADIS INTERNATIONAL LTD, vol. 58, no. 3, 1999, pages 492-496, XP000874675 ISSN: 0012-6667
- GRAUL A ET AL: "TEGASEROD MALEATE" DRUGS OF THE FUTURE,ES,BARCELONA, vol. 24, no. 1, 1999, pages 38-44, XP000874672 ISSN: 0377-8282

## Description

The present invention relates to a pharmaceutical composition, in particular to a composition for administering active agents which are poorly soluble in aqueous media and/or which are acid sensitive. More particularly, the present invention relates to a pharmaceutical composition for administering active agents acting on the gastro-intestinal system. The present invention also relates to a process for manufacturing such compositions. The term "pharmaceutical" also covers veterinary use.

Pharmaceutical compositions containing active agents which are poorly soluble in aqueous media and/or acid sensitive are difficult to manufacture. One of the problems that may occur concerns adsorption of the active agent on the process equipment during the manufacturing process. Due to the poor solubility of such active agents it is also difficult to obtain pharmaceutical compositions which upon administration have a good dissolution rate. As a further problem, active agents may be degraded, e.g., chemically, during a manufacturing process using acidic conditions or during the storage of the composition.

The present invention provides compositions and processes which avoids or minimise one or more of the above problems.

We have now surprisingly found that it is possible to produce a pharmaceutical composition for administering of active agents which are poorly soluble in aqueous media, e.g., pure water, and/or acid sensitive, and which upon administration has good dissolution properties, a good bioavailability and is surprisingly efficacious.

The present invention provides in one aspect a solid oral pharmaceutical composition, e.g., a tablet, comprising Tegaserod or a pharmaceutically acceptable salt, and a disintegrant, e.g., a super-disintegrant, which is present in an amount of at least 15% by weight based on the total weight of the composition.

By "acid sensitive" is meant an active agent which under even slightly acidic conditions, e.g., at pH 6, may be transformed to a significant extent in a degradation product, e.g., by chemical degradation, which may have no or changed activity, e.g., within 2 hours. Examples of compounds are known in the art and may be ascertained by routine experimentation.

By "disintegrant" is meant a substance or mixture of substance added to a solid pharmaceutical composition, e.g., a tablet, to facilitate its break-up or disintegration after administration in order that the active ingredient is released from the composition as efficiently as possible to allow for its rapid dissolution (see e.g. "Remington's Pharmaceutical Science" 18th edition (1990), "The Theory and Practice of Industrial Pharmacy" Lachman et al. Lea & Febiger (1970)).

We have also found difficulties on producing stable commercially acceptable formulations, e.g., tablets, of compounds such as those disclosed in EP505322 and which are useful as 5-HT₄ receptor agonists or partial agonists.

Tegaserod (3-(5-methoxy-1 H-indol-3-yl-methylene)-N-pentylcarbazimidamide) (example 13) of formula 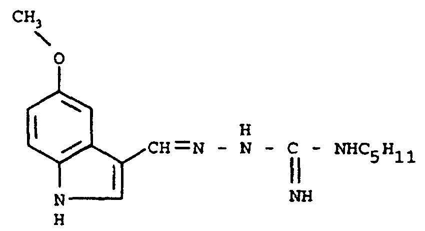 or a pharmaceutically acceptable salt form thereof, e.g., the hydrogen maleate (hereinafter "hml") salt is referred hereinafter as Compound A. Compound A has a solubility of about 0.02% at 25°C in water and is acid sensitive. We have found that compositions may be produced which give good absorption even in the stomach. We have also found that Compound A may be adsorbed by certain excipients so that its dissolution upon administration may be substantially reduced.

Little has been published in detail on 5-HT₄ receptor agonists, partial agonists or antagonists biopharmaceutical properties, e.g., their site of action is not known.

The present invention provides in a further aspect pharmaceutical compositions allowing a complete dissolution of Compound A, when administered to humans, e.g., patients, in need thereof. These compositions allow a good bioavailability and are surprisingly efficacious. Moreover, they are stable and well reproducible. A process for their preparation is also provided.

The 5-HT₄ receptor is a cloned species of the serotonin receptor family which comprises at least 14 distinct G protein-coupled receptors (the receptor ionophore of the 5-HT₃ subtype excluded). Four splice variants of the human receptor, 5-HT_{4A}, 5-HT_{4B}, 5-HT_{4C}, and 5-HT_{4D}, have been identified which differ in the length and sequence of the protein's C terminus (Blondel et al., FEBS Letters (1997) 412:465-474; Blondel et al., J. Neurochem. (1998) 70: 2252-2261). Biochemical characterisation of 5-HT₄ receptors revealed a positive coupling to adenylyl cyclase. 5-HT₄ receptor expression in man has been found in the brain, the gut, the atria, the urinary bladder and kidneys.

Compounds capable of acting on the serotonin receptor are substituted benzamides, e.g., cisapride, renzapride, zacopride, clebopride, cinitapride, mosapride, lintopride, metoclopramide, or benzoic esters, e.g., RS 23597-190, SB 204070, SB 207710, or aminoguanidines, zacopride, prucalopride, SB 205149, SC 53116, RS 67333, RS 67506, BIMU 1, BIMU 8, (S)-RS 56532, Tropisetron, Alosetron, GR 113808, GR 125487, SB 207266, RS 23597, RS 39604, RS 100235, DAU 6285, SC 53606, 3-(5-hydroxy-7-methyl-1H-indol-3-yl- methylene)-N-pentyl-N-methyl-carbazimidamide, indazole-3-carboxamides, 2-oxobenzamidazole-3-carboxamides (as disclosed in EP 908 459) etc.

5-HT₄ receptor agonists are considered as compounds which can activate 5-HT₄ receptors under quiescent/resting conditions (complete or partial activation). As 5-HT₄ receptor full agonists or partial agonists one may cite (S)-zacopride, cisapride, prucalopride, SB 205149, SC 53116, RS 67333, RS 67506, BIMU 1, BIMU 8, (S)-RS 56532 and Compound A, particularly its hydrogen maleate salt.

5-HT₄ receptor antagonists are considered as compounds which do not activate 5-HT₄ receptors but act as inhibitors of agonists at 5-HT₄ receptors. As 5-HT₄ receptor antagonists one may cite GR 113808, GR 125487, SB 203186, SB 204070, SB 207266, RS 23597, RS 39604, RS 100235, DAU 6285, SC 53606, 3-(5-hydroxy-7-methyl-1H-indol-3-ylmethylene)-N-pentyl-N-methyl-carbazimidamide.

5-HT₄ receptor agonists are useful for the prevention and treatment of gastro-intestinal motility disorders, e.g., Irritable Bowel Syndrome (IBS), Gastro-Esophageal Reflux Disease (GERD), Functional Dyspepsia (FD) and Post Operative Ileus (POI).

In a preferred embodiment, the composition of the invention comprises 20 to 60%, e.g., 30 to 50%, e.g. 40% by weight of disintegrant based on the total weight of the composition. We have observed that the use of such a high percentage of disintegrant further improves the dissolution rate in aqueous media, but also prevents the active agent from adsorbing on excipients.

As disintegrants the composition of the invention may comprise :
- crospovidone (molecular weight >10⁶), e.g., Polyplasdone XL®, Kollidon CL®, Polyplasdone XL-10®,
- pregelatinised starch (MW : 30 000 - 120 000), e.g., starch 1500®, STA-Rx 1500®,
- sodium starch glycolate (MW : 500 000 - 1 000 000), e.g. Primojel®,
- carboxymethylcellulose calcium (CMC-Ca),
- carboxymethylcellulose sodium (CMC-Na) (MW: 90 000 - 700 000), e.g., Ac-Di-Sol®,
- sodium alginate,

or a mixture thereof.

Preferably, the disintegrant is crospovidone which is preferably water insoluble. Preferably it rapidly exhibits high capillary or pronounced hydration capacity with little tendency to gel formation. Preferably the particle size is from about 1 to 500 micrometers. Preferred particle size distribution is less than 400 micrometers, e.g., for Polyplasdone XL®, less than 80 micrometers, e.g., less than 74 micrometers for, e.g., Polyplasdone XL-10®, approximately 50% greater than 50 micrometers and maximum of 1% greater than 250 micrometers in size for, e.g., Kollidon CL®. A preferred crospovidone is Polyplasdone XL®, e.g., with a density of about 0.213 g/cm³ (bulk) or 0.273 g/cm³ (tapped).

The pharmaceutical composition of the invention may further comprise one or more excipients.

The composition may further comprise one or more lubricants, e.g., in an amount within the range of from, e.g., 1 to 20%, e.g., from 5 to 15%, e.g., 10% by weight of the composition.

Examples of such lubricants include
- glyceryl mono fatty acid, e.g., having a molecular weight of from 200 to 800, e.g., glyceryl monostearate (e.g., Myvaplex®, USP quality)
- polyethylene glycol (PEG), having a molecular weight of from 100 to 10000, e.g., 1000 to 8000, e.g., 2000 to 6000, e.g., 2500 to 5000, e.g., Macrogol 4000 (Pulver) BP,
- hydrogenated castor oil (e.g., Cutina®), and the like

or a mixture thereof.

In a preferred composition the lubricant is glyceryl monostearate. The lubricant properties of such preferred composition may be improved by adding polyethylene glycol (PEG), e.g., Macrogol 4000 (Pulver) BP.

The composition of the invention may comprise one or more surfactants, e.g., in an amount in the range of from 0.1 to 10%, e.g., 1 to 5%, e.g. 2% by weight of the total composition. Pharmaceutically suitable surfactants may be non-ionic or anionic.

As non-ionic surfactants one may use:
- polyoxyethylene-sorbitan-fatty acid esters (polysorbates; MW: 500 to 2000), e.g., mono- and tri- lauryl, palmityl, stearyl and oleyl esters, e.g., Tween®, e.g., Tween 80®;
- polyoxyethylene fatty acid esters (MW : 500 to 5000), e.g., Myrj® or Cetiol®;
- polyoxyethylene-polyoxypropylene co-polymers, e.g., having a molecular weight of from 1000 to 20 000, e.g., 6 000 to 15 000, e.g., 7 000 to 10 000, e.g., Pluronic® or Emkalyx®;
- polyoxyethylene-polyoxypropylene block co-polymers e.g., having a molecular weight of from 1000 to 20 000, e.g., 6 000 to 15 000, e.g., 7 000 to 10 000, e.g., Poloxamer 188®;
- reaction products of a natural or hydrogenated castor oil and ethylene oxide, e.g., Cremophor®;
- dioctylsuccinate or di-[2-ethylhexyl]- succinate;
- propyleneglycol mono- and di-fatty acid (e.g. C₆-C₈) esters, e.g., Miglyol®;

or mixtures thereof.

As suitable anionic surfactants one may use, e.g., sodium laurylsulfate or docusate sodium.

Unless where otherwise stated fatty acid or carbon containing chain is from about 8 to 22 carbon atoms, e.g., C₁₈.

The composition of the invention may comprise one or more binders, e.g., in an amount in the range of from 1 to 10%, e.g., 2 to 8%, e.g., 5% by weight. One may particularly use :
- hydroxypropylmethylcellulose, e.g., having a molecular weight of from 10 000 to 1 500 000, e.g., HPMC-3 (3mPa-s) (e.g. Pharmacoat®, Methocel®),
- polyvinylpyrrolidone, e.g., having a molecular weight of from 2500 to 3 000 000, e.g., 8 000 to 1 000 000, e.g., 10 000 to 400 000, e.g., 30 000 to 50 000 (e.g., Kollidon®, Plasdone®),
- potato starch, wheat starch, com starch, e.g., having a molecular weight of from 30 000 to 120 000,

or a mixture thereof.

The composition of the invention may comprise one or more diluents such as lactose, mannitol, sucrose, calcium sulphate, calcium phosphate, microcristalline cellulose (Avicel®) in an amount within the range of from, e.g., 10 to 70%, e.g., 20 to 50%, e.g., 30% by weight of the composition. Preferably, the diluent is lactose, e.g., lactose 200 mesh (e.g., from DMV® or Alpavit ®), e.g., the monohydrated form.

Other conventional excipients which may optionally be present in the composition of the invention include preservatives, stabilisers, anti-adherents or silica flow conditioners or glidants, e.g., silicon dioxide (e.g., Syloid®, Aerosil®) as well as FD&C colours such as ferric oxides.

Other excipients disclosed in the literature, as for instance in Fiedler's "Lexicon der Hilfstoffe", 4th Edition, ECV Aulendorf 1996 and "Handbook of Pharmaceutical Excipients" Wade and Weller Ed.(1994), the contents of which are incorporated herein by reference, may be used in the pharmaceutical compositions according to the invention.

The invention is particularly useful for pharmaceutical compositions containing an active agent, e.g., an 5HT₄ receptor agonist, partial agonist or antagonist, e.g., compound A, e.g., the hydrogen maleate salt, which is present in an amount within the range of from about 0.2% to about 20%, e.g. 0.5 to 15%, and preferably from about 1% to about 10% by weight of the composition.

A preferred composition of the invention may comprise from about 0.5 to about 15% by weight of compound A, e.g., the hydrogen maleate salt, from 20 to 60% by weight of disintegrant, e.g., crospovidone, from 1 to about 20% by weight of a lubricant, e.g., monoglycerylstearate, from 0.1 to about 10% by weight of a surfactant, e.g., poloxalkol, from about 10 to 50% by weight of a diluent, e.g., lactose, and from 1 to 10% by weight of a binder, e.g., hydroxypropylmethyl cellulose (e.g. HPMC-3). From 1 to 10% by weight of PEG may also be added.

The weight ratio of the active agent to the disintegrant may be from 1:1 to 1:400, e.g., 1:5 to 1:100, 1:8 to 1:50, e.g., 1:16 to 1:20.

In a further aspect the present invention provides a pharmaceutical oral, e.g., tablet, - composition comprising one of the active agents cited above, e.g., a 5-HT₄ agonist, partial agonist or antagonist, e.g., Tegaserod, said composition having dissolution characteristics in water or in USP buffers pH 6.8 and 7.5 of:

| time (minutes) | amount (percentage) |
|---|---|
| 5 | 30 - 90 |
| 15 | 80 - 100 |
| 30 | 95 - 100 |
| 60 | 100 |

For example a composition according to the invention, e.g., comprising Tegaserod as the active agent, may have dissolution characteristics in water or in USP buffers pH 6.8 and 7.5 of:

| time (minutes) | amount (percentage) |
|---|---|
| 5 | 48.9 |
| 15 | 95.5 |
| 30 | 99.7 |
| 60 | 100 |

In a further aspect the present invention provides a pharmaceutical oral, e.g., tablet, composition comprising Tegaserod, wherein in use 80% of said active agent is released in water or in USP buffers pH 6.8 and 7.5 within 5 minutes.

In a further aspect, the present invention provides the use of at least 15% by weight of a disintegrant in the manufacturing of pharmaceutical composition for the administration of compound A, e.g. the hydrogen maleate salt.

The pharmaceutical compositions of the present invention are useful in the known indications of the particular active agent incorporated therein.

The exact amounts of active agent and of the formulation to be administered depend on a number of factors, e.g. the condition to be treated, the desired duration of treatment and the rate of release of active agent.

For example, the amount of the active agent required and the release rate thereof may be determined on the basis of conventional in vitro or in vivo techniques, determining how long a particular active agent concentration in the blood plasma remains at an acceptable level for a therapeutic effect.

Examples of doses provided in a solid formulation, e.g., a tablet, are, for Irritable Bowel Syndrome (IBS), 1 mg to 12 mg of active agent, for functional dyspepsia (FD) and gastroesophageal reflux disease (GERD), 0.2 to 2mg of compound A, e.g. the hydrogen maleate salt, per day for a 70 kilogram mammal, e.g. humans, and in standard animal models. The increased tolerability of compound A, e.g. the hydrogen maleate salt, provided by the compositions may be observed in standard animal tests and in clinical trials.

The pharmaceutical composition of the invention comprising compound A is particularly useful for improving sensory perception of rectal distension, e.g. for the treatment of anal incontinence, or for preventing, modulating or treating visceral pain or discomfort.

5-HT₄ receptor agonists, partial agonists or antagonists, e.g. as disclosed in EP-A1-505,322, on the basis of observed activity, e.g. stimulatory effect on the peristaltic reflex in the isolated guinea-pig ileum, e.g. as described in EP-A1-505,322, have been found to be useful for the treatment of gastro-intestinal motility disorders, for example to normalise or to improve the gastric emptying and intestinal transit in subjects having a disturbed motility, e.g. in irritable bowel syndrome.

In accordance with the present invention, it has now surprisingly been found that compound A has a beneficial effect, e.g. it exerts modulating effects, on the sensory perception of rectal distension and on visceral sensitivity or perception.

It is admitted that receptor properties are not uniform throughout the gut and that the type of afferent innervation reflects the quality of sensations originating from a particular organ. For example, the rectum belongs to those parts of the gastro-intestinal tract from which also non-painful sensations arise, in contrast to the colon from which only painful sensations emanate.

Anal incontinence may be due to functional disturbances of the main anal continence mechanisms. Anal continence appears to be based on a co-ordinated functioning of the neuromuscular machinery managing rectal sensation and compliance, the recto-anal inhibitory reflex, reflex contractions of the external anal sphincter and the puborectalis muscle. Although skeletal muscle (external sphincter and puborectalis) contractions are of great importance in the maintenance of continence, it is probably the triggering effect of rectal sensation and perception that plays a crucial role and, in fact, is frequently abnormal in incontinent patients. Anal incontinence is a dysfunction which occurs particularly in diabetics and the elderly population.

There is a medical need for modulating visceral sensitivity, discomfort or pain in patients suffering from gastro-intestinal disorders and for a treatment of anal continence dysfunctions.

In accordance with the particular findings of the present invention, there is provided:
1.1. A method for preventing, modulating or treating visceral, e.g. abdominal, pain or discomfort in a subject in need thereof, which method comprises administering to said subject an effective amount of Compound A or a pharmaceutically acceptable salt thereof.
1.2. A method for modulating visceral sensitivity or perception in a subject in need thereof, which method comprises administering to said subject an effective amount of Compound A or a pharmaceutically acceptable salt thereof.
1.3. A method for stimulating 5-HT₄ receptors present on afferent nerve terminals, particularly on extrinsic neurones of the gut, in a subject in need thereof, which method comprises administering to said subject an effective amount of Compound A or a pharmaceutically acceptable salt thereof.
1.4. A method for modulating visceral sensitivity, discomfort or pain via stimulation of 5-HT₄ receptors present on afferent nerve terminals, particularly on extrinsic neurones of the gut, in a subject in need thereof, which method comprises administering to said subject an effective amount of compound A or a pharmaceutically acceptable salt thereof.
1.5. A method for regulating or stabilising myenteric plexus-afferent fibbers in a subject in need thereof, which method comprises administering to said subject an effective amount of compound A or a pharmaceutically acceptable salt thereof.
1.6. A method for improving sensory perception of rectal distension in a subject in need thereof, which method comprises administering to said subject an effective amount of Compound A or a pharmaceutically acceptable salt thereof.
1.7. A method for treating anal continence dysfunctions in a subject in need thereof, which method comprises administering to said subject an effective amount of Compound A or a pharmaceutically acceptable salt thereof.

As alternative to the above the present invention also provides:
2. A pharmaceutical composition for use in a method as defined under 1.1 to 1.7 above comprising Compound A or a pharmaceutically acceptable salt thereof, together with one or more pharmaceutically acceptable diluents or carriers therefor, e.g. a composition such as disclosed hereinabove.

Utility of compound A in the prevention, modulation or treatment of visceral, *e.g*., abdominal pain or discomfort or modulation of visceral sensitivity or perception or regulation or stabilisation of myenteric plexus-afferent fibers, is demonstrated in convenient tests, e.g., in accordance with the method hereinafter described.

Decerebrate, anaesthesia-free cats under continuous monitoring of blood pressure are paralysed by alcuronium chloride dissolved in rheomacrodex i.v. (200 µg/kg initially and supplementary doses of 100 µg/kg, if necessary), and artificially ventilated. Single unit activity of afferent fibres are recorded in a monopolar fashion from peripheral endings of centrally cut filaments of sacral dorsal roots. Tension receptors are identified by probing of their receptive fields in the wall of the mobilised rectum. Thereafter, the response of the units to barostat -controlled rectal ramp-distension is determined. The quantitative response characteristics of the units is evaluated with respect to distension pressure and resulting rectal diameter. Alternatively, the response of the units to pressure-induced peristalsis is measured.

After obtaining 2 distension profiles (5 min each) and/or 10 min of peristalsis under control conditions, a 5-HT₄ receptor agonist, partial agonist or antagonist, *e.g*., Compound A, or vehicle is applied i.v. and the protocol is repeated. Subsequently, the activity of additional units is recorded in the presence of a 5-HT₄ receptor agonist, partial agonist or antagonist, *e.g*., Compound A, or vehicle according to the distension/peristalsis protocol. In this assay, the firing rate of the rectal afferents is reduced after administration of a 5-HT₄ receptor agonist or partial agonist at a dose range of from 0.1 to 3 mg/kg i.v., at distension pressures above 20 mmHg. With Compound A, administered i.v. in incremental doses from 0.15 to 1.2 mg/kg, the most prominent inhibition occurs at 50 mmHg and a half-maximal reduction is obtained at about 0.7 mg/kg.

Utility of Compound A, in the treatment of anal incontinence as well as utility in treating conditions as hereinabove specified, may be demonstrated in accordance with the method hereinafter described.

Intraluminal pressures and reflexes in the last 60 cm of the colon of 10 fasted healthy volunteers are measured by means of perfusion manometry. Three latex balloons positioned at 50, 30 and 10 cm, allow volume stimulation. Basal values of colonic intraluminal pressures and reflexes are established. Subsequently, reflex inhibitory relaxations of the internal anal sphincter is triggered by inflating the balloons by 10 ml increments up to a maximum volume of 150 ml. During the inflation phase, two parameters are evaluated: a) the reflux threshold (volume able to induce a substantial pressure decrease of the internal anal sphincter); and b) the sensation threshold (volume able to induce a conscious defecation reflex). After the basal recordings, each subject is given a 5-HT₄ receptor agonist, partial agonist or antagonist, *e.g*., Compound A, p.o. and 30 to 90 min later the colonic intraluminal pressure and reflexes are assessed again by the same method. In this test, the 5-HT₄ receptor agonist, partial agonist or antagonist, *e.g*., Compound A, significantly reduced the sensation threshold when administered at a dose of 2-12 mg p.o.

Compound A, may be administered by any conventional route, in particular enterally, preferably orally, *e.g*., in the form of tablets or capsules, or parenterally, *e.g*., in the form of injectable solutions or suspensions or in a suppository form.

Compound A, may be administered in free form or in pharmaceutically salt form. Such salts exhibit the same order of activity as the 5-HT₄ receptor agonists, partial agonists or antagonists in free form.

Daily dosages required in practising the method of the present invention will vary depending upon, for example, the particular compound employed, the mode of administration and the severity of the condition to be treated. An indicated daily dose is in the range of from about 0.05 to about 30 mg, *e.g*., from about 0.05 to about 5 mg for parenteral use, and of from about 0.1 to about 30 mg for oral use, conveniently administered once or in divided dosages 2 to 4x/day, or in sustained release form. Unit dosage forms for oral administration accordingly comprise from about 0.5 to about 30 mg of 5-HT₄ receptor agonist, partial agonist or antagonist, *e.g*., Compound A, or a pharmaceutically acceptable salt thereof, admixed with an appropriate solid or liquid, pharmaceutically acceptable diluent or carrier therefor.

Furthermore, it has also been found that Compound A, has a beneficial effect in the prevention or treatment of gastro-intestinal motility disorders, e.g. a stimulatory effect on gastrointestinal motility, in horses and cattle.

Accordingly, there is also provided:
3.1. A method for preventing or treating gastro-intestinal motility disorders, e.g. by - stimulating the motility of the gastro-intestinal tract in horses or cattle in need thereof, which method comprises administering to the horses or cattle an effective amount of Compound A, or a pharmaceutically acceptable salt thereof.
3.2. A method for preventing or treating gastro-intestinal motility disorders, e.g. after colic surgery, e.g. post-operative lleus, in horses or cattle in need thereof, which method comprises administering to the horses or cattle an effective amount of Compound A, or a pharmaceutically acceptable salt thereof.
4. A pharmaceutical composition for veterinary use, e.g. in horses or cattle, e.g. in any of the method 5.1. or 5.2. as indicated above, comprising Compound A, or a pharmaceutically acceptable salt thereof, together with a pharmaceutically acceptable diluent or carrier therefor, e.g. a composition as disclosed hereinabove.

Utility of Compound A, in the treatment of post-operative lleus as well as utility in treating conditions as hereinabove specified in horses or cattle, may be demonstrated in accordance with the method hereinafter described.

20 horses having colic syndrome are submitted to abdominal surgery. During surgery supportive therapy is applied to them. At the end of surgery, Compound A, is administered i.v. or i.m., e.g. at a dose of from 0.01 to 10 mg/kg. This dose is repeated every 8 to 24 h until spontaneous defecation is observed. Gastro-intestinal motility is evaluated based e.g. on the presence or absence of gastric reflux as determined by nasogastric intubation, occurrence of borborygmi and timing of defecation after the first injection of the test compound. In this test, Compound A, is effective in restoring normal motility function of the equine intestine.

Daily dosages required in practising the veterinary method of the present invention will vary depending upon, for example, the particular compound employed, the mode of administration and the severity of the condition to be treated. An indicated daily dose is in the range of from about 0.01 to about 10 mg/kg, *e.g*., from about 0.05 to about 5 mg/kg for parenteral use, conveniently administered once or in divided dosages 2 to 4x/day, or in sustained release form.

In a further aspect the invention provides a method for preventing or treating gastro-intestinal motility disorders in a subject, e.g., a human or an animal, in need of such a therapy comprising administering to this subject an effective amount of a composition according to the present invention.

In a further aspect the invention a process is provided for improving dissolution properties in aqueous media of a pharmaceutical composition containing compound A, e.g. the hydrogen maleate salt.

The pharmaceutical composition of the invention may be prepared by any conventional method known in the art, e.g., by mixing an appropriate amount of Compound A, with at least 15%, e.g., from 20 to 60%, e.g., from 30 to 50%, e.g., 40%, by weight of a disintegrant based on the total weight of the composition.

It is preferred to formulate in solid form, e.g., unit dosage form. Typical form include capsules and preferably compressed forms such as tablets.

The pharmaceutical composition according to the invention may be prepared by e.g., a wet, e.g., water based, granulation manufacturing process (the process equipment, as glass material, may be pre-treated with a siliconizing agent) comprising the successive steps of :
i) pre-mixing compound A, e.g. the hydrogen maleate salt with 60 to 98% of the diluent, and then sieving the resulting mixture,
ii) mixing purified water with the binder in a weight ratio of from 1:20 to 3:20, and stirring until dissolution,
iii) adding the surfactant to the solution of ii) and stirring until dissolution,
iv) adding the disintegrant, the remaining diluent and 50 to 70% of the first lubricant to the pre mixture of i) and mixing
v) wetting the mixture of step iv) with the granulating solution from step iii) while mixing
vi) granulating the mixture of step v) by mixing,
vii) drying the granulate to reach a required loss on drying, e.g., for the tabletting mixture
viii) sizing the granulate by sieving.

For tablet manufacturing the granulate from viii) is mixed, e.g., in a free fall mixer, with the rest of the first lubricant and the second one to obtain the desired final tabletting mixture which may be compressed into tablets. This may be performed with conventional tabletting machines on, e.g., a rotary machine, at compression pressures of, e.g., 2 to 30 KN, e.g. 5 to 27 KN, e.g., 10 to 20 KN (KN = Kilo Newtons).

The composition according to the invention may also be prepared by an alternative wet granulation manufacturing process wherein the pre-mixing and sieving of step i) are not performed. In this case, the active agent, the disintegrant, the diluent and about 60% of the first lubricant are pre-blended together and then wetted with the wetting solution of step iv).

Compositions comprising any of the above-mentioned active agents may be prepared by a process as disclosed above.

If desired the pharmaceutical compositions of the invention are stored under low relative humidity conditions, e.g., rH (relative humidity) less than 50%, e.g., below e.g. 30-50%, and at room temperature, preferably less than 20°C. The compositions provide storage stable systems. Insignificant degradation is detected after storage of up to 1 year at room temperature, e.g., 25 °C.

The compositions of the invention may be packed in conventional manner to keep out humidity, e.g., in a blister pack, optionally with a desiccant.

The compositions of the invention may have a water content of from 0 to 3% based on the total weight of the composition.

The present invention relates in a further aspect to a composition, in particular comprising compound A, as obtained by one of the above processes to provide a small, stable form.

### Examples

The following examples illustrate the manufacturing, on an industrial scale, of compositions comprising compound A hml using a wet granulation process as disclosed above.

### Example 1

A 2 mg tablet formulation may be prepared as described hereinafter.

### a) Preparation of the granulated material

### Premixing step

1. 4.432 kg of compound A hml and 28.688 kg of lactose monohydrate are mixed with an intensive mixer (Colette Gral® 300 I or Fielder®) ; mixer speed setting : 1; chopper speed setting : 1) for approximately 1.5 minutes, or with a free fall mixer (Turbula®, Soneco® or Röhnrad®)
2. The pre-mixture from step 1 is then sieved (oscillating granulator, e.g., Frewitt® or Erweka®; mesh size: 0.8 millimetres).
3. The pre-mixture is divided into two portions of 16.560 kg.

### Preparation of the granulating solution

4. Approximately 40 kg of purified water are weighed out.
5. 3.600 kg of methylhydroxypropylcellulose 3 maps are added to the purified water from step 4 and this is stirred until dissolution.
6. 1.440 kg of poloxamer 188 are added to the solution from step 5 while stirring until dissolution.

### Granulating step

7. 28.800 kg of crospovidone, 10.080 kg of lactose monohydrate and 4.320 kg of glyceryl monostearate are weighed out.
8. One portion of the premixture from step 3 is added to the excipients from step 7 and this is mixed with the intensive mixer, e.g., Colette Gral® 300 I or Fielder® (mixer speed setting : 1; chopper speed setting : 1) for approximately 2 minutes.
9. The mixture from step 8 is wetted with the granulating solution from step 6 while mixing with the intensive mixer, e.g., Colette Gral® 300 l or Fielder® (mixer speed setting : 1; chopper speed setting : 0; pumping rate approximately : 4 kg/minute) for approximately 12 minutes.
10. Approximately 2 kg of purified water are weighed out.
11. The vessel from step 6 is rinsed with the purified water from step 10 and this is added to the mixture from step 9 while mixing.
12. The mass is granulated by mixing with the intensive mixer, Colette Gral® 300 I or Fielder® (mixer speed setting : 1; chopper speed setting : 1) for approximately 2.5 minutes.

### Drying step

13. The granulate from step 12 is dried in a fluidised air bed drier (e.g., Glatt® or Aeromatic®) for approximately 65 minutes (inlet air temperature approximately 70°C) to reach the required loss on drying (LOD) for the tabletting mixture, i.e., until LOD ≤4.4%.
14. The granulate sized by sieving (0.8 millimetres) with an oscillating sieve granulator, e.g., Frewitt® or Erweka®.
15. Steps 4 to 14 are repeated with the other portion of step 3.

### b) Preparation of the tabletting mixture

16. 8.640 kg of polyethylene glycol 4000 and 5.760 kg of glyceryl monostearate are sieved (oscillating granulator, e.g., Frewitt® or Erweka®; mesh size: 0.8 millimetres)
17. The ingredients from step 16 are added to the total mass of granulated material and this is mixed with a free fall mixer, e.g., Soneco® or Röhnrad®, for approximately 20 minutes (10 rpm) to obtain the desired final tabletting mixture.

### c) Compression step

18. The tabletting mixture from step 17 is pressed into tablets using compression pressures of 11, 14 or 17 KN on a rotary tabletting machine, e.g., Fette®, Korsh®, Kelian® or Coarty® (temperature < 20°C; rH (relative humidity) < 40%)

### Example 2 : Composition of a 2 mg tablet (1 mg of base corresponds to 1.385 mg of the hydrogen maleate salt of compound A)

| | |
|---|---|
| Compound A hml | 2.77 (2mg base) |
| Polyplasdone XL USP/NF | 36.00 |
| Glyceryl monostearate USP/NF | 9.00 |
| Poloxalkol | 1.80 |
| Lactose 200 mesh | 30.53 |
| HPMC 3cPs | 4.50 |
| Polyethyleneglycol 4000 | 5.40 |
| Water adsorbed | 2.00 |
| Total | 92 mg |

### Example 3

A 6 mg tablet formulation may be prepared by the manufacturing process described hereinafter.

### a) Preparation of the granulated material

### Preparation of the granulating solution

1. Approximately 40 kg of purified water are weighed out.
2. 3.600 kg of methylhydroxypropylcellulose 3 maps are added to the purified water from step 1 while stirring until dissolution.
3. 1.440 kg of poloxamer 188 are added to the solution from step 2 while stirring until dissolution (mixing tank under stirring).

### Granulating step

4. 4.787 kg of compound A hml and 28.800 kg of crospovidone, 21.853 kg of lactose monohydrate and 4.320 kg of glyceryl monostearate are weighed out.
5. The ingredients from step 4 are mixed with the intensive mixer, e.g., Colette Gral® 300 I or Fielder® (mixer speed setting: 1; chopper speed setting : 1) for approximately 2 minutes.
6. The mixture from step 5 is wetted with the granulating solution from step 3 while mixing with the intensive mixer, e.g., Colette Gral® 300 I or Fielder® (mixer speed setting: 1; chopper speed setting : 0; pumping rate approximately 4 kg/minute) for approximately 12 minutes.
7. Approximately 2 kg of purified water are weighed out.
8. The vessel from step 3 is rinsed with the purified water from step 7 and this is added to the mixture from step 6 while mixing.
9. The mass is granulated by mixing with the intensive mixer, e.g., Colette Gral® 300 I or Fielder® (mixer speed setting: 1; chopper speed setting : 1) for approximately 2.5 minutes.

### Drying step

10. The granulate from step 9 is dried in a fluidised air bed drier, e.g., Glatt® or Aeromatic®) for approximately 65 minutes (Inlet air temperature approximately 70°C) to reach the desired loss on drying (LOD) for the tabletting mixture, i.e., until LOD ≤4,4%.
11. The granulate sized by sieving (0.8 millimetres) with an oscillating-sieve granulator (Frewitt® or Erweka®)
12. Steps 1 to 11 are repeated.

### b) Preparation of the tabletting mixture

13. 8.640 kg of polyethylene glycol 4000 and 5.760 kg of glyceryl monostearate are sieved with an oscillating sieve granulator, e.g., Frewitt® or Erweka® (0.8 millimetres)
14. The ingredients from step 13 are added to the total mass of granulated material and this is mixed with a free fall mixer, e.g., Soneca® or Rohnrad®, for approximately 20 minutes (10 rpm) in the desired final tabletting mixture.

### c) Compression step

15. The tabletting mixture from step 14 is pressed into tablets using compression pressures of 13, 16 or 19 KN on a rotary tabletting machine, e.g., Fette®, Korsh®, Kelian® or Coarty® (temperature<20°C, rH (relative humidity) < 40 %).

### Example 4 : Composition of a 6 mg tablet (1 mg of base corresponds to 1.385 mg of hydrogen maleate of compound A):

| | |
|---|---|
| Compound A hml | 8.31 (6mg base) |
| Polyplasdone XL USP/NF | 50.00 |
| Glyceryl monostearate USP/NF | 12.50 |
| Poloxalkol | 2.50 |
| Lactose 200 mesh | 37.94 |
| HPMC 3cPs | 6.25 |
| Polyethyleneglycol 4000 | 7.50 |
| Water adsorbed | 3.00 |
| Total | 128 mg |

### Example 5

A 0.5 mg tablet formulation may be prepared by the manufacturing process described hereinafter.

### a) Preparation of the granulated material

### Premixing step

1. 1.994 kg of compound A hml and 31.126 kg of lactose monohydrate are mixed with an intensive mixer (Colette Gral® 300 I or Fielder®) ; mixer speed setting : 1; chopper speed setting : 1) for approximately 1.5 minutes, or with a free fall mixer (Turbula® Soneco® or Röhnrad®)
2. The premixture from step 1 is then sieved (oscillating granulator, e.g., Frewitt® or Erweka®; mesh size: 0.8 millimetres).
3. The premixture is divided into two portions of 16.560 kg.

### Preparation of the granulating solution

4. Approximately 43 kg of purified water are weighed out.
5. 3.600 kg of methylhydroxypropylcellulose 3 maps are added to the purified water from step 4 and this is stirred until dissolution.
6. 1.440 kg of poloxamer 188 are added to the solution from step 5 while stirring until dissolution.

### Granulating step

7. 28.800 kg of crospovidone, 10.080 kg of lactose monohydrate and 4.320 kg of glyceryl monostearate are weighed out.
8. One portion of the premixture from step 3 is added to the excipients from step 7 and this is mixed with the intensive mixer, e.g., Colette Gral® 300 l or Fielder® (mixer speed setting : 1; chopper speed setting : 1) for approximately 2 minutes.
9. The mixture from step 8 is wetted with the granulating solution from step 6 while mixing with the intensive mixer, e.g., Colette Grat®300 I or Fielder® (mixer speed setting : 1; chopper speed setting : 0; pumping rate approximately : 4 kg/minute) for approximately 12 minutes.
10. Approximately 2 kg of purified water are weighed out.
11. The vessel from step 6 is rinsed with the purified water from step 10 and this is added to the mixture from step 9 while mixing.
12. The mass is granulated by mixing with the intensive mixer, Colette Gral® 300 I or Fielder® (mixer speed setting : 1; chopper speed setting : 1) for approximately 2.5 minutes.

### Drying step

13. The granulate from step 12 is dried in a fluidised air bed drier (e.g., Glatt® or Aeromatic®) for approximately 60 minutes (inlet air temperature approximately 70°C) to reach the required loss on drying (LOD) for the tabletting mixture, i.e. until LOD ≤4,5%.
14. The granulate sized by sieving (0.8 millimetres) with an oscillating sieve granulator, e.g., Frewitt® or Erweka®.
15. Steps 4 to 14 are repeated with the other portion of step 3.

### b) Preparation of the tabletting mixture

16. 8.640 kg of polyethylene glycol 4000 and 5.760 kg of glyceryl monostearate are sieved (oscillating granulator, e.g., Frewitt® or Erweka®; mesh size: 0.8 millimetres)
17. The ingredients from step 16 are added to the total mass of granulated material and this is mixed with a free fall mixer, e.g., Soneco® or Rohnrad®, for approximately 20 minutes (10 rpm) to obtain the desired final tabletting mixture.

### c) Compression step

18. The tabletting mixture from step 17 is pressed into tablets on a rotary tabletting machine, e.g., Fetter Korsh®, Kelian® or Coarty® (temperature < 20°C; rH (relative humidity) < 40%)

### Example 6 : Composition of a 0.5 mg tablet (1 mg of base corresponds to 1.385 mg of the hydrogen maleate salt of compound A)

| | |
|---|---|
| Compound A hml | 0.6925 (0.5mg base) |
| Polyplasdone XL USP/NF | 20.00 |
| Glyceryl monostearate USP/NF | 5.00 |
| Poloxalkol | 1.00 |
| Lactose 200 mesh | 17.8075 |
| HPMC 3cPs | 2.50 |
| Polyethyleneglycol 4000 | 3.00 |
| Water adsorbed | 1.00 |
| Total | 51 mg |

### Example 7 : Composition of a 12 mg tablet (1 mg of base corresponds to 1.385 mg of the hydrogen maleate salt of compound A)

The manufacturing process is similar to the process used for the 6mg tablets.

| | |
|---|---|
| Compound A hml | 16.62 (12mg base) |
| Polyplasdone XL USP/NF | 72.00 |
| Glyceryl monostearate USP/NF | 18.00 |
| Poloxalkol | 3.60 |
| Lactose 200 mesh | 49.98 |
| HPMC 3cPs | 9.0 |
| Polyethyleneglycol 4000 | 10.8 |
| Water adsorbed | 4.00 |
| Total | 184 mg |

## Claims

1. A solid oral pharmaceutical composition comprising Compound A 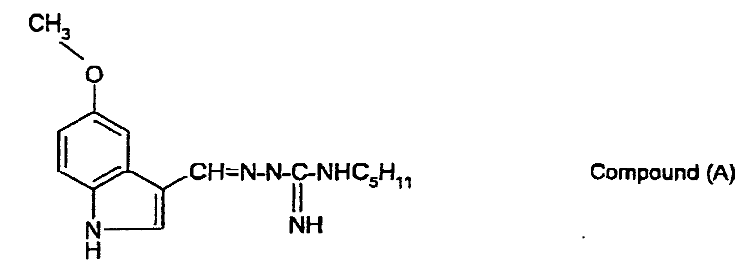
or its salts; comprising a disintegrant which is present in an amount of at least 15% by weight based on the total weight of the composition, wherein the disintegrant is a member selected from the group consisting of crospovidone, pregelatinised starch, sodium starch glycolate, carboxymethylcellulose sodium, carboxymethylcellulose calcium, sodium alignate, and a mixture thereof.

2. The composition of claim 1 wherein Compound A is in the form of its hydrogen maleate salt.

3. The composition of claims 1 or 2 wherein the disintegrant is crospovidone.

4. The composition according to claims 1, 2 or 3 **characterized in that** the disintegrant is present in an amount of 15 to 60% by weight based on the total weight of the composition.

5. The composition according to claims 1, 2 or 3 **characterized in that** the disintegrant is present in an amount of 15 to 40% by weight based on the total weight of the composition.

6. The composition according to claims 1, 2 or 3 **characterized in that** the disintegrant is present in an amount of 20 to 60% by weight based on the total weight of the composition.

7. The composition according to claims 1, 2 or 3 **characterized in that** the disintegrant is present in an amount of 20 to 40% by weight based on the total weight of the composition.

8. The composition according to claim 3 **characterized in that** the disintegrant is present in an amount of 39 to 40% by weight based on the total weight of the composition.

9. The composition according to claim 3 **characterized in that** the disintegrant is present in an amount of 39,2% by weight based on the total weight of the composition.

10. The composition according to claim 3 **characterized in that** the disintegrant is present in an amount of 39,1% by weight based on the total weight of the composition.

11. The composition according to claims 3 **characterized in that** the disintegrant is present in an amount of 39,0% by weight based on the total weight of the composition.

12. The composition according to claims 1, 2 or 3 **characterized in that** the disintegrant is present In an amount of 40% by weight based on the total weight of the composition.

13. The composition according to claims 1, 2 or 3 comprising a lubricant.

14. The composition according to claims 1, 2 or 3 comprises a lubricant which is a glyceryl mono fatty acid.

15. The composition according to claims 1, 2 or 3 comprises a lubricant which is a mixture of glyceryl monostearate and polyethylene glycol.

16. The composition according to claims 1, 2 or 3 comprising a surfactant.

17. The composition according to claims 1, 2 or 3 comprising a surfactant which is poloxamer.

18. The composition according to claims 1, 2 or 3 wherein said composition is a tablet

19. Use of a composition according to claims 1, 2 or 3 for the manufacture of a medicament for preventing, modulating or treating visceral pain or discomfort, for modulating visceral sensitivity or perception, for improving sensory perception of rectal distension, or for treating anal continence dysfunctions in a subject in need thereof.

20. Use of a composition according to claims 1, 2 or 3 for the manufacture of a medicament for preventing or treating gastro-intestinal motility disorders in horses or cattle in need thereof.

## Patentansprüche

1. Feste orale pharmazeutische Zusammensetzung mit einem Gehalt an der Verbindung A 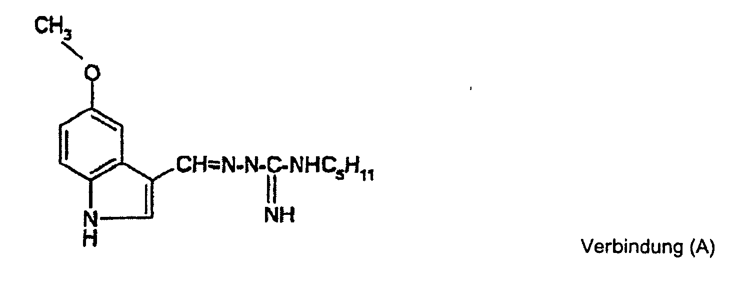 oder einem Salz hiervon, **dadurch gekennzeichnet, dass** sie ein Zerfallhilfsmittel enthält, das in einer Menge von wenigstens 15 Gew.-%, bezogen auf das Gesamtgewicht der Zusammensetzung, vorhanden ist, wobei dieses Zerfallhilfsmittel ein Stoff ist, der aus der Gruppe ausgewählt ist, welche besteht aus Crospovidon, vorgelatinisierter Stärke, Natriumstärkeglycolat, Carboxymethylcellulosenatrium, Carboxymethylcellulosecalcium, Natriumalginat und einem Gemisch hiervon.

2. Zusammensetzung nach Anspruch 1, worin die Verbindung A in Form ihres Hydrogenmaleatsalzes vorliegt.

3. Zusammensetzung nach Anspruch 1 oder 2, worin das Zerfallhilfsmittel Crospovidon ist.

4. Zusammensetzung nach Anspruch 1, 2 oder 3, **dadurch gekennzeichnet, dass** das Zerfallhilfsmittel in einer Menge von 15 bis 60 Gew.%, bezogen auf das Gesamtgewicht der Zusammensetzung, vorhanden ist.

5. Zusammensetzung nach Anspruch 1, 2 oder 3, **dadurch gekennzeichnet, dass** das Zerfallhiffsmittel in einer Menge von 15 bis 40 Gew.-%, bezogen auf das Gesamtgewicht der Zusammensetzung, vorhanden ist.

6. Zusammensetzung nach Anspruch 1, 2 oder 3, **dadurch gekennzeichnet, dass** das Zerfallhilfsmittel in einer Menge von 20 bis 60 Gew.-%, bezogen auf das Gesamtgewicht der Zusammensetzung, vorhanden ist.

7. Zusammensetzung nach Anspruch 1, 2 oder 3, **dadurch gekennzeichnet, dass** das Zerfallhilfsmittel in einer Menge von 20 bis 40 Gew.-%, bezogen auf das Gesamtgewicht der Zusammensetzung, vorhanden ist.

8. Zusammensetzung nach Anspruch 3, **dadurch gekennzeichnet, dass** das Zerfallhilfsmittel in einer Menge von 39 bis 40 Gew.-%, bezogen auf das Gesamtgewicht der Zusammensetzung, vorhanden ist.

9. Zusammensetzung nach Anspruch 3, **dadurch gekennzeichnet, dass** das Zerfallhilfsmittel in einer Menge von 39,2 Gew.-%, bezogen auf das Gesamtgewicht der Zusammensetzung, vorhanden ist.

10. Zusammensetzung nach Anspruch 3, **dadurch gekennzeichnet, dass** das Zerfallhilfsmittel in einer Menge von 39,1 Gew.-%, bezogen auf das Gesamtgewicht der Zusammensetzung, vorhanden ist.

11. Zusammensetzung nach Anspruch 3, **dadurch gekennzeichnet, dass** das Zerfallhilfsmittel in einer Menge von 39,0 Gew.-%, bezogen auf das Gesamtgewicht der Zusammensetzung, vorhanden ist.

12. Zusammensetzung nach Anspruch 1, 2 oder 3, **dadurch gekennzeichnet, dass** das Zertallhilfsmittel in einer Menge von 40 Gew.-%, bezogen auf das Gesamtgewicht der Zusammensetzung, vorhanden ist.

13. Zusammensetzung nach Anspruch 1, 2 oder 3, die ein Schmiermittel enthält.

14. Zusammensetzung nach Anspruch 1, 2 oder 3, die ein Schmiermittel enthält, das eine Glycerylmonofettsäure ist.

15. Zusammensetzung nach Anspruch 1, 2 oder 3, die ein Schmiermittel enthält, das ein Gemisch aus Glycerylmonostearat und Polyethylenglycol ist.

16. Zusammensetzung nach Anspruch 1, 2 oder 3, die ein Tensid enthält.

17. Zusammensetzung nach Anspruch 1, 2 oder 3, die ein Tensid enthält, welches ein Poloxamer ist.

18. Zusammensetzung nach Anspruch 1, 2 oder 3, worin diese Zusammensetzung eine Tablette ist.

19. Verwendung einer Zusammensetzung nach Anspruch 1, 2 oder 3 zur Herstellung eines Arzneimittels zur Prävention, Modulation oder Behandlung von Viszeralschmerz oder Viszeralbeschwerden, zur Modulation von Viszeralsensitivität oder Viszeralperzeption, zur Verbesserung einer sensorischen Perzeption von Rektaldistension oder zur Behandlung von Analkontinenzdysfunktionen bei einem behandlungsbedürftigen Subjekt.

20. Zusammensetzung nach Anspruch 1, 2 oder 3 zur Herstellung eines Arzneimittels zur Prävention oder Behandlung von Gastrointestinalmotilitätsstörungen bei behandlungsbedürftigen Pferden oder Rindern.

## Revendications

1. Composition pharmaceutique solide destinée à une administration par voie orale comprenant un composé A 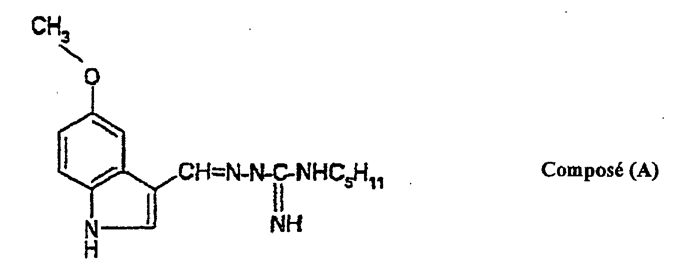
ou ses sels ; comprenant un délitant présent selon une quantité d'au moins 15% en poids sur base du poids total de la composition, dans laquelle le délitant est un membre sélectionné à partir du groupe composé de crospovidone, d'amidon pré-gélatiné, de glycolate d'amidon de sodium, de carboxyméthylcellulose de sodium, de carboxyméthylcellulose de calcium, d'alginate de sodium et de mélange de ceux-ci.

2. Composition selon la revendication 1 **caractérisé en ce que** le composé A est présent sous forme de son sel de maléate d'hydrogène.

3. Composition selon la revendication 1 ou 2 **caractérisé en ce que** le délitant est du crospovidone.

4. Composition selon les revendications 1, 2 ou 3 **caractérisé en ce que** le délitant est présent selon une quantité allant de 15 à 60% en poids sur base du poids total de la composition.

5. Composition selon les revendications 1, 2 ou 3 **caractérisé en ce que** le délitant est présent selon une quantité allant de 15 à 40% en poids sur base du poids total de la composition.

6. Composition selon les revendications 1, 2 ou 3 **caractérisé en ce que** le délitant est présent selon une quantité allant de 20 à 60% en poids sur base du poids total de la composition.

7. Composition selon les revendications 1, 2 ou 3 **caractérisé en ce que** le délitant est présent selon une quantité allant de 20 à 40% en poids sur base du poids total de la composition.

8. Composition selon la revendication 3 **caractérisé en ce que** le délitant est présent selon une quantité allant de 39 à 40% en poids sur base du poids total de la composition.

9. Composition selon la revendication 3 **caractérisé en ce que** le délitant est présent selon une quantité allant de 39,2% en poids sur base du poids total de la composition.

10. Composition selon la revendication 3 **caractérisé en ce que** le délitant est présent selon une quantité allant de 39,1% en poids sur base du poids total de la composition.

11. Composition selon la revendication 3 **caractérisé en ce que** le délitant est présent selon une quantité allant de 39,0% en poids sur base du poids total de la composition.

12. Composition selon les revendications 1, 2 ou 3 **caractérisé en ce que** le délitant est présent selon une quantité allant de 40% en poids sur base du poids total de la composition.

13. Composition selon les revendications 1, 2 ou 3 comprenant un lubrifiant.

14. Composition selon les revendications 1, 2 ou 3 comprenant un lubrifiant qui est un acide gras mono-glycéryle.

15. Composition selon les revendications 1, 2 ou 3 comprenant un lubrifiant qui est un mélange de monostéarate de glycéryle et de polyéthylèneglycol.

16. Composition selon les revendications 1, 2 ou 3 comprenant un agent tensioactif.

17. Composition selon les revendications 1, 2 ou 3 comprenant un agent tensioactif qui est un poloxamère.

18. Composition selon les revendications 1, 2 ou 3 **caractérisée en ce que** ladite composition est un comprimé.

19. Composition selon les revendications 1, 2 ou 3 pour la fabrication d'un médicament permettant de prévenir, moduler ou le traiter une douleur ou un inconfort viscéral, de moduler la sensibilité ou la perception viscérale, d'améliorer la perception sensitive de la dystémie rectale ou de traiter les dysfonctionnements de continence anale chez un sujet nécessitant un tel traitement.

20. Utilisation d'une composition selon les revendications 1, 2 ou 3 pour la fabrication d'un médicament pour la prévention ou le traitement de troubles liés à la motilité gastro-intestinales chez les chevaux ou le bétail.
